# EUROPEAN PATENT APPLICATION

(11) **EP 4 806 371 A1**
(43) Date of publication of application: **16.09.2026**
(21) Application number: 24899355.2
(22) Date of filing: 28.08.2024
(51) Int. Cl.: A61B 17/12, A61B 17/3207

(54) **TISSUE LIGATION SYSTEM**

(30) Priority: 07.12.2023 CN 202311666136
(71) Applicant: Shanghai Cingular Biotech Corporation, Shanghai 200120 (CN)
(72) Inventor: JIANG, Wei, Shanghai 200120 (CN); YANG, Jun, Shanghai 200120 (CN); ZHANG, Li, Shanghai 200120 (CN); MAO, Shaowen, Shanghai 200120 (CN); TAN, Haiying, Shanghai 200120 (CN)
(74) Representative: Regimbeau
(86) International application number: PCT/CN2024/115210
(87) International publication number: WO 2025/118709

(57) **Abstract**

A tissue ligation system includes a mesh sleeve structure (2) and a ligation device (1). The mesh sleeve structure (2) includes a mesh pouch (21), a base sleeve (22), and a waist sleeve (23). The base sleeve (22) is circumferentially connected to an open end of the mesh pouch (21). The waist sleeve (23) is arranged circumferentially along a waist of the mesh pouch (21). The ligation device (1) includes a connecting rod (16) and a ligation mechanism. The ligation mechanism includes a handle housing (11), a pushing assembly (12), a first tightening assembly (13), and a second tightening assembly (14). The first tightening assembly (13) includes a first fixed rod (161), a first movable rod (162), and a first driving assembly. The second tightening assembly (14) includes a second fixed rod (163), a second movable rod (164), and a second driving assembly. The base sleeve (22) includes a base tightening tether (223). Two ends of the base tightening tether (223) are connected to the first fixed rod (161) and the first movable rod (162), respectively. The first driving assembly is connected to and drives the first movable rod (162) to move, thereby driving the base tightening tether (223) to tighten. The waist sleeve (23) includes a waist tightening tether (231). Two ends of the waist tightening tether (231) are connected to the second fixed rod (163) and the second movable rod (164), respectively. The second driving assembly is connected to and drives the second movable rod (164) to move, thereby driving the waist tightening tether (231) to tighten. The system can contract and squeeze to expel excess blood inside a neoplastic object or soft tissue, thereby preventing secondary thrombosis, accelerating atrophy and endothelialization of the neoplastic object or soft tissue, and promoting postoperative recovery.

## Description

This application claims priority to Chinese Patent Application No. 202311666136.1 filed with the China National Intellectual Property Administration (CNIPA) on Dec. 07, 2023, the disclosure of which is incorporated herein by reference in its entirety.

### TECHNICAL FIELD

This application relates to a medical device, for example, a tissue ligation system.

### BACKGROUND

Surgeries are generally performed to resect most neoplasms or redundant soft tissues in a human body, which usually achieves a good therapeutic effect. However, for some neoplasms or soft tissues that are not suitable for resection, such as some hemangiomas and the left atrial appendage, ligation or occlusion is often the only available means for treatment. Most ligation or occlusion products on the market are surgical medical devices, which need to be operated through a surgical operation, resulting in relatively large trauma and a long postoperative recovery time.

With the rapid development of interventional medical technology, transcatheter products have emerged. One category is occlusion devices, such as the Watchman system, the Lambre system, and the WaveCrest system. These products enter the left atrium through transseptal puncture and implant an occluder at the base of the left atrial appendage, which requires high operational skills of an operator and poses a risk of causing secondary thrombosis.

Another category is clipping devices, including clip-type devices and snare-type ligation devices. The clip-type devices, such as the AtriClip series from Atricure and the E-Clip series from Beijing Meditech Medical Technology Co., Ltd., involve delivering a clip in an open state to encircle or clamp the base of the left atrial appendage through a surgical operation and then withdrawing a delivery device so that the clip is closed to close the left atrial appendage. The operation is relatively simple; however, the clip has a fixed shape and a constant clamping force. Since human left atrial appendages vary greatly due to individual differences, the adaptability of such clips is poor. The snare-type ligation devices, such as the LARIAT system from SentreHeart, include an epicardial snare device and a transseptal magnetic attraction device for positioning and capturing the left atrial appendage. The base of the left atrial appendage is ligated through cooperation of the two devices. The operation is relatively cumbersome and also carries the risk of secondary thrombosis. For another example, the Sierra system from Aegis includes an epicardial snare device and a positioning and capture device, which requires positioning prior to ligating the base with a snare.

In addition, none of the various interventional ligation and occlusion products mentioned above, whether internal occlusion or external ligation, can evacuate excess blood inside a tissue, resulting in a risk of thrombosis. Therefore, there is an urgent need to develop an interventional ligation and occlusion product that is both simple and easy to operate and capable of expelling excess blood inside the tissue.

### SUMMARY

A tissue ligation system includes a mesh sleeve structure and a ligation device.

The mesh sleeve structure includes a mesh pouch with one or two open ends and a base sleeve. The mesh pouch is configured to surround a target tissue. The base sleeve is circumferentially connected to an open end of the mesh pouch and is configured to tighten the base of the target tissue. In addition, at least one waist sleeve is arranged circumferentially at the waist of the mesh pouch to contract and squeeze excess blood inside the target tissue.

The ligation device includes a connecting rod and a ligation mechanism. One end of the connecting rod is connected to the ligation mechanism, and the other end of the connecting rod is connected to the mesh sleeve structure through a tether inside the connecting rod. The ligation mechanism includes a handle housing, a pushing assembly, and a tightening assembly. The pushing assembly and the tightening assembly are fixed inside the handle housing after being nested and combined. The tightening assembly includes a first tightening assembly connected to the base sleeve and a second tightening assembly connected to the waist sleeve.

In an optional embodiment, the pushing assembly includes an outer sheath tube, an inner catheter, a sliding member, and a pushing button.

The outer sheath tube and the inner catheter are mounted at a front end of the handle housing. One end of the sliding member is connected to the outer sheath tube, and the other end of the sliding member is connected to the pushing button. A slide rail groove is disposed on the handle housing. The pushing button is slidably disposed inside the slide rail groove.

In an optional embodiment, the mesh pouch is woven from a polymer or metal material having elasticity or a shape memory property.

In an optional embodiment, the mesh pouch is disposed at a front end of an inner lumen of the outer sheath tube. A rear end of the outer sheath tube is connected to a front end of the sliding member. A rear end of the sliding member is connected to the pushing button. Backward or forward movement of the pushing button drives the sliding member to drive the outer sheath tube to move backward or forward, thereby releasing or retracting the mesh pouch.

In an optional embodiment, the sliding member is connected to the pushing button through a round shaft. A front end of the sliding member is a connecting sleeve, and a rear end of the sliding member is a connecting block. The connecting sleeve is connected to a rear end of the outer sheath tube. The connecting block protrudes from the surface of the connecting sleeve and forms a height difference. The bottom of the connecting block is connected to and integrally formed with the connecting sleeve. The top of the connecting block is connected to a front end of the round shaft. The pushing button is connected to a rear end of the round shaft.

In an optional embodiment, the first tightening assembly includes a first fixed rod, a first movable rod, a guide rail slider, a fixed guide rail, a threaded guide sleeve, and a tail knob.

The base sleeve includes a base tightening tether. Two ends of the base tightening tether are connected to the first fixed rod and the first movable rod, respectively. The first fixed rod is fixed to a front end of the handle housing. The first movable rod is connected to the guide rail slider. The guide rail slider is slidably disposed in the fixed guide rail. The fixed guide rail is fixedly mounted inside the handle housing through a concave-convex structure. A threaded guide sleeve is sleeved outside the fixed guide rail. The guide rail slider is provided with an external thread. The threaded guide sleeve is provided with an internal thread. The guide rail slider is driven to move axially in a groove on the fixed guide rail through rotation of the threaded guide sleeve. The tail knob is fixedly connected to the threaded guide sleeve. Rotation of the tail knob drives the threaded guide sleeve to rotate.

Movement of the guide rail slider drives the first movable rod to move, thereby driving the base tightening tether to tighten the base of the mesh pouch.

In an optional embodiment, the first tightening assembly also includes a multi-stage telescopic rod. Every two adjacent telescopic rods of the multi-stage telescopic rod are fitted through concave-convex engagement, and four degrees of freedom including up, down, left, and right between the telescopic rods are limited. The telescopic rods extend and retract forward and backward relative to each other. The foremost stage telescopic rod is connected to the guide rail slider. Forward and backward movement of the guide rail slider drives the multi-stage telescopic rod to extend and retract.

In an optional embodiment, the fixed guide rail includes an upper fixed block and a lower fixed block. The upper fixed block and the lower fixed block are connected via a buckle. Front and rear ends of each of the upper fixed block and the lower fixed block are connected to the housing through a concave-convex fit.

In an optional embodiment, the second tightening assembly includes a second fixed rod, a second movable rod, and a wire retraction component. The waist sleeve includes a waist tightening tether. Two ends of the waist tightening tether are connected to the second fixed rod and the second movable rod, respectively. The second fixed rod is fixed to a front end of the handle housing. The second movable rod passes through the handle housing and is connected to the wire retraction component. The wire retraction component is connected to the waist tightening tether.

In an optional embodiment, a threading hole and a cutting edge are formed at a front end of each of the first fixed rod, the second fixed rod, the first movable rod, and the second movable rod. Threading holes are connected to the base tightening tether and the waist tightening tether.

In an optional embodiment, the wire retraction component includes a Luer connector. The Luer connector is connected to a rear end of the handle housing. One end of the waist tightening tether connected to the second movable rod is connected to the Luer connector.

In an optional embodiment, the mesh pouch is a woven polygonal structure and a three-dimensional open structure with an internal space. An opening is disposed at an upper end of the mesh pouch, or an opening is disposed at each of an upper end and a lower end of the mesh pouch. The base sleeve is circumferentially connected to the opening at the upper end of the mesh pouch.

In an optional embodiment, the base sleeve consists of a partially non-closed cavity portion having a certain rigidity, an elongated connecting portion, and a base tightening tether. The partially non-closed cavity portion is a C-shaped or U-shaped rigid cavity, or a semi-arc cavity. One end of the elongated connecting portion is connected to the middle of the cavity portion, and another portion of the elongated connecting portion is fixed to a front end of the inner catheter. One or multiple strands of the base tightening tether extending from the upper end of the mesh pouch are stored inside the cavity portion of the base sleeve. The base tightening tether is made of the same material as the material used for weaving the mesh pouch. During a tightening process, the base tightening tether extending from the upper end of the mesh pouch gradually disengages from the interior of the cavity portion along a non-closed portion of the cavity portion of the base sleeve.

In an optional embodiment, at least one lumen is disposed inside the ligation device. The lumen allows entry of an optical fiber lens to facilitate a surgical field of view and also allows injection of contrast agent through the lumen.

A method of using the preceding tissue ligation system includes steps (a) to (g).
(a) A connecting rod at a front end of a ligation device is introduced into a body via a catheter sheath through percutaneous puncture and is delivered to the vicinity of a neoplastic object or soft tissue to be ligated, such as a hemangioma or a left atrial appendage.
(b) A pushing button is moved to drive an outer sheath tube to retract, thereby releasing a mesh sleeve structure.
(c) The entire ligation device is pushed forward until a mesh pouch completely covers the neoplastic object or soft tissue such that a position of a base sleeve is substantially flush with a base of the neoplastic object or soft tissue.
(d) A tail knob is rotated to drive a guide rail slider and a first movable rod to retract and tighten a tether inside the base sleeve, thereby gradually contracting and reducing the base diameter of the neoplastic object or soft tissue.
(e) After the base sleeve is partially tightened, the tail-end knob of a Luer connector is pulled outward to tighten a waist sleeve and squeeze excess blood inside the left atrial appendage.
(f) The base tightening tether inside the base sleeve continues to be tightened until the base tightening tether sufficiently binds the base of the neoplastic object or soft tissue and completely closes the base.
(g) The entire ligation device is rotated, the tether is severed by a cutting edge at the front end of the connecting rod, and the entire ligation device is then withdrawn.

### BRIEF DESCRIPTION OF DRAWINGS

FIG. 1 is a view illustrating the overall structure of a tissue ligation system according to this application.
FIG. 2 is a perspective view of a ligation device according to an embodiment of this application.
FIG. 3 is an exploded view of a handle housing according to an embodiment of this application.
FIG. 4 is an exploded view of a pushing assembly according to an embodiment of this application.
FIG. 5 is a view illustrating the connection of various parts of a pushing assembly according to an embodiment of this application.
FIG. 6 is a view illustrating the structure of a sliding member according to an embodiment of this application.
FIG. 7 is an exploded view of a first tightening assembly according to an embodiment of this application.
FIG. 8 is a sectional structural view illustrating the working principle of a first tightening assembly according to an embodiment of this application.
FIG. 9 is a view illustrating the structure of a second tightening assembly according to an embodiment of this application.
FIG. 10 is a view illustrating the structure of a front end portion of each metal rod according to an embodiment of this application.
FIG. 11 is an overall exploded view of a ligation device according to an embodiment of this application.
FIG. 12 is a view illustrating the structure of a mesh sleeve structure with one open end according to an embodiment of this application.
FIG. 13 is a view illustrating the structure of a mesh sleeve structure with two open ends according to an embodiment of this application.
FIGS. 14A, 14B, and 14C are views illustrating the structure of a mesh pouch in different states according to an embodiment of this application.
FIG. 15 is a view illustrating the connection relationship between a sleeve and a mesh pouch according to an embodiment of this application.
FIG. 16 is a view illustrating the working state of a tissue ligation system according to an embodiment of this application.
FIG. 17 is a view illustrating the working principle of a mesh sleeve structure ligating tissue according to an embodiment of this application.
FIG. 18 is a diagram illustrating the steps of a method of using a tissue ligation system according to an embodiment of this application.

### Reference list

- 1: ligation device
- 11: handle housing
- 111: upper housing
- 112: lower housing
- 113: buckle
- 114: slot
- 115: slide rail groove
- 116: limiting groove
- 12: pushing assembly
- 121: outer sheath tube
- 122: sliding member
- 1221: connecting sleeve
- 1222: connecting block
- 1223: round shaft
- 123: pushing button
- 124: inner catheter
- 1241: lead hole
- 13: first tightening assembly
- 131: guide rail slider
- 1311: external thread
- 132: first telescopic rod
- 133: second telescopic rod
- 134: third telescopic rod
- 135: fourth telescopic rod
- 136: fixed guide rail
- 1361: upper fixed block
- 1362: lower fixed block
- 1363: groove
- 137: threaded guide sleeve
- 1371: internal thread
- 138: tail knob
- 14: second tightening assembly
- 141: Luer connector
- 1411: tail-end knob
- 15: plug
- 16: connecting rod
- 161: first fixed rod
- 162: first movable rod
- 163: second fixed rod
- 164: second movable rod
- 165: threading hole
- 166: cutting edge
- 2: mesh sleeve structure
- 21: mesh pouch
- 211: rope loop
- 22: base sleeve
- 221: cavity portion
- 222: elongated connecting portion
- 223: base tightening tether
- 23: waist sleeve
- 231: waist tightening tether
- 24: front end opening
- 25: rear end opening
- 3: heart
- 31: left atrial appendage

### DETAILED DESCRIPTION

Embodiments of this application are described in detail below. Examples of the embodiments are illustrated in the drawings, where the same or similar reference numerals indicate the same or similar elements or elements having the same or similar functions. The embodiments described below with reference to the drawings are illustrative, only for explaining this application and not to be construed as limiting this application.

In the description of this application, it is to be understood that the orientation or position relationships indicated by terms "center", "longitudinal", "lateral", "length", "width", "thickness", "above", "below", "front", "back", "left", "right", "vertical", "horizontal", "top", "bottom", "inside", "outside", "clockwise", and "counterclockwise", are based on the orientation or position relationships shown in the drawings, only for facilitating the description of this application and simplifying the description, and do not indicate or imply that the device or element referred to has a specific orientation and is constructed and operated in a specific orientation, and thus it is not to be construed as limiting this application. Moreover, terms "first" and "second" are used only for the purpose of description and are not to be construed as indicating or implying relative importance or implicitly indicating the number of technical features indicated. Thus, a feature defined as a "first" feature or a "second" feature may explicitly or implicitly include one or more of this feature. As used herein, the term "plurality" is defined as two or more, unless otherwise expressly specified and limited.

In the description of this application, it is to be noted that unless otherwise expressly specified and limited, terms "mounted", "connected to each other", and "connected" are to be construed in a broad sense, for example, as permanently connected, detachably connected, or integratedly connected; mechanically connected, electrically connected, or a connection enabling mutual communication; directly connected or indirectly connected via an intermediate medium; or internally communicated of the two elements or the interaction between the two elements. For those of ordinary skill in the art, specific meanings of the preceding terms in this application may be construed according to specific circumstances.

In this application, unless otherwise expressly specified and limited, when a first feature is described as "above" or "below" a second feature, the first feature and the second feature may be in direct contact or be in contact via another feature between the two features instead of being in direct contact. Moreover, when the first feature is "on", "above", or "over" the second feature, the first feature is right on, above, or over the second feature, or the first feature is obliquely on, above, or over the second feature, or the first feature is simply at a higher level than the second feature. When the first feature is "under", "below", or "underneath" the second feature, the first feature is right under, below, or underneath the second feature, or the first feature is obliquely under, below, or underneath the second feature, or the first feature is simply at a lower level than the second feature.

The description below provides many different embodiments or examples for implementing different structures of this application. To simplify the description of this application, components and configurations of particular examples will be described below, which are, of course, illustrative only and are not intended to limit this application. Moreover, this application may repeat reference numbers and/or reference letters in different examples. Such repetition is for the purpose of simplification and clarity, and does not indicate a relationship between the discussed various embodiments and/or arrangements. In addition, this application provides examples of various specific processes and materials, but those of ordinary skill in the art can conceive the application of other processes and/or the use of other materials.

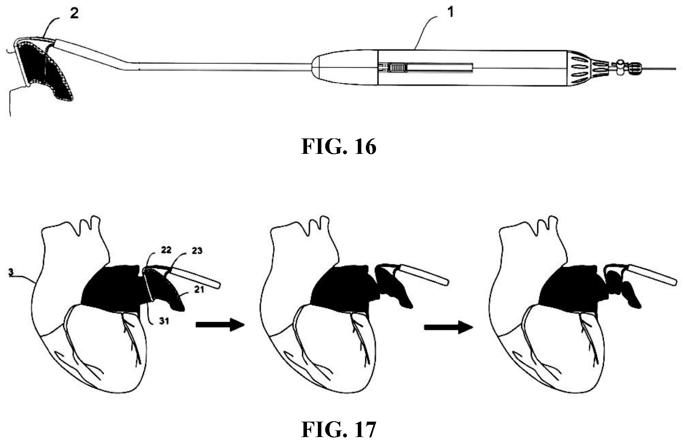

The embodiments of this application described below illustrate the application device in a form suitable for ligating the left atrial appendage 31 of a human heart 3 (see FIG. 17). It is to be understood that the following embodiments discuss the use at the left atrial appendage 31. However, unless otherwise noted, variations of the system, device, and method are not limited to the use for ligation of the left atrial appendage 31. Rather, this application may be used for ligating any neoplastic object or soft tissue in a body. Moreover, this application is applicable to different parts of the human body that are similar to the left atrial appendage 31. In addition, this application may be used in various procedures where the benefits of the method and/or device are desired.

Unless otherwise specified, all technical and scientific terms used in this application have the same meaning as commonly understood by a person of ordinary skill in the art to which this disclosure belongs. It is also be understood that terms defined in commonly used dictionaries should be interpreted as having meanings consistent with the meanings in the context of the relevant field, and should not be interpreted in an idealized or overly formal sense unless clearly defined otherwise.

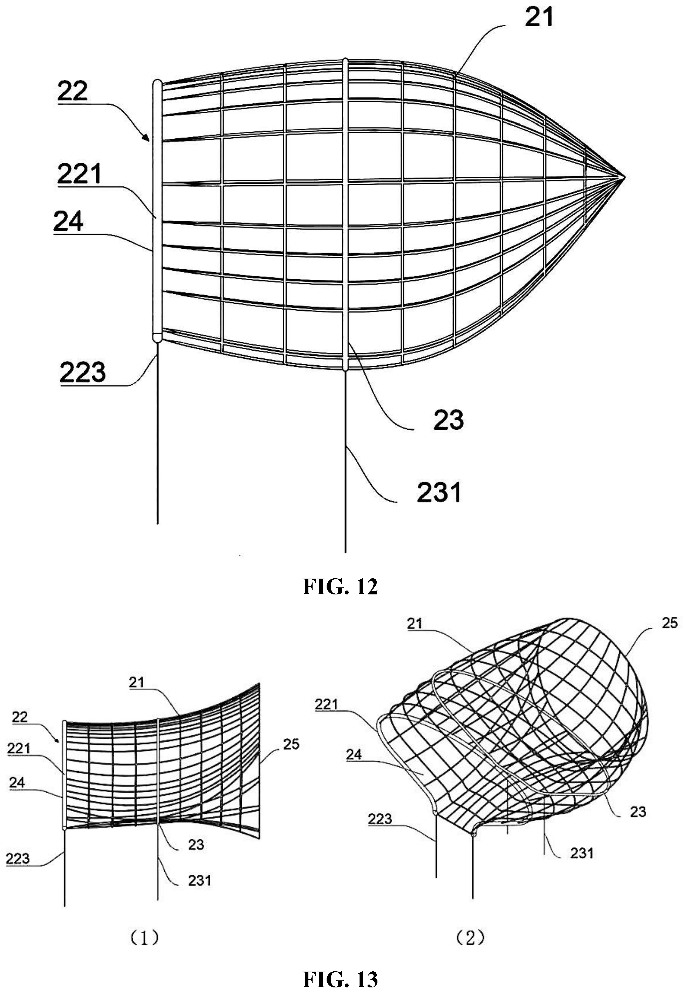

For ease of description, the "front end" in this application refers to an end away from an operator, and the "rear end" refers to an end close to the operator. The "front end" of tissue in this application refers to an end close to the ligation device 1. The "base" of tissue refers to an end away from the ligation device 1. The "waist" or "middle" of tissue refers to the entire area between the front end and the base of the tissue. The "waist" or "middle" of the mesh pouch 21 (see FIG. 12) refers to the entire area between the two ends of the mesh pouch 21.

The left atrial appendage 31 is an important structure inside the heart 3 and is often associated with heart 3 diseases and stroke. When excess blood accumulates inside the left atrial appendage 31, thrombosis may occur, thereby increasing the risk of stroke. Therefore, the development of an effective method to ligate the left atrial appendage 31 and expel excess blood carries important clinical significance.

In view of the problems that conventional interventional tissue ligation devices pose a risk of causing secondary thrombosis, or that the operation requiring auxiliary instruments or systems to position prior to ligation is cumbersome and time-consuming, a tissue ligation system is designed in this application. The tissue ligation system does not require transendocardial septal puncture, nor does the tissue ligation system require an additionally configured instrument to position and capture tissue, making the operation simpler. While ligating the base of the tissue, the tissue ligation system can also squeeze excess blood inside the tissue, accelerate tissue atrophy and endothelialization, and promote postoperative recovery.

The technical solutions of this application are described hereinafter in conjunction with drawings and embodiments.

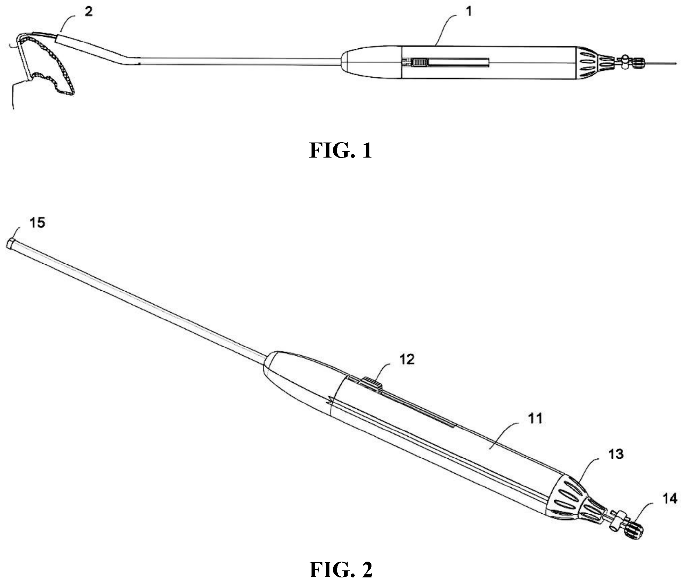

In an embodiment provided by this application, the tissue ligation system provided by this application is an innovative implantable medical device for treating diseases related to the heart 3. Specifically, the device ligates a neoplastic object or soft tissue in a body, such as a left atrial appendage, and expels excess blood inside the tissue. For example, the device addresses issues inside the left atrial appendage 31 to reduce or prevent accumulation of excess blood. The system includes a mesh sleeve structure 2, a ligation device 1 (see FIG. 1), and corresponding components, and may be used through specific steps. The components of the system, the method of use, and features of the system will be described in this embodiment.

### Ligation system

With reference to FIG. 1, the tissue ligation system includes a mesh sleeve structure 2 and a ligation device 1. The mesh sleeve structure 2 serves as a covering and tightening component for covering and ligating the left atrial appendage 31 and squeezing excess blood inside the left atrial appendage 31.

The ligation device 1 is configured to release the mesh sleeve structure 2, deliver the mesh sleeve structure 2 to the outer edge of the left atrial appendage 31, and tighten the mesh sleeve structure 2.

Regarding the ligation device 1, with reference to FIG. 2, the ligation device 1 as a whole in this application includes a handle housing 11, a pushing assembly 12, a first tightening assembly 13, and a second tightening assembly 14. The pushing assembly 12 is configured to release the mesh sleeve structure 2 and deliver the mesh sleeve structure 2 to the left atrial appendage 31. The first tightening assembly 13 is connected to and tightens a base tightening tether 223 inside a cavity portion 221 of a base sleeve 22 of the mesh sleeve structure 2. The second tightening assembly 14 is connected to and tightens a waist tightening tether 231 of a waist sleeve 23 of the mesh sleeve structure 2.

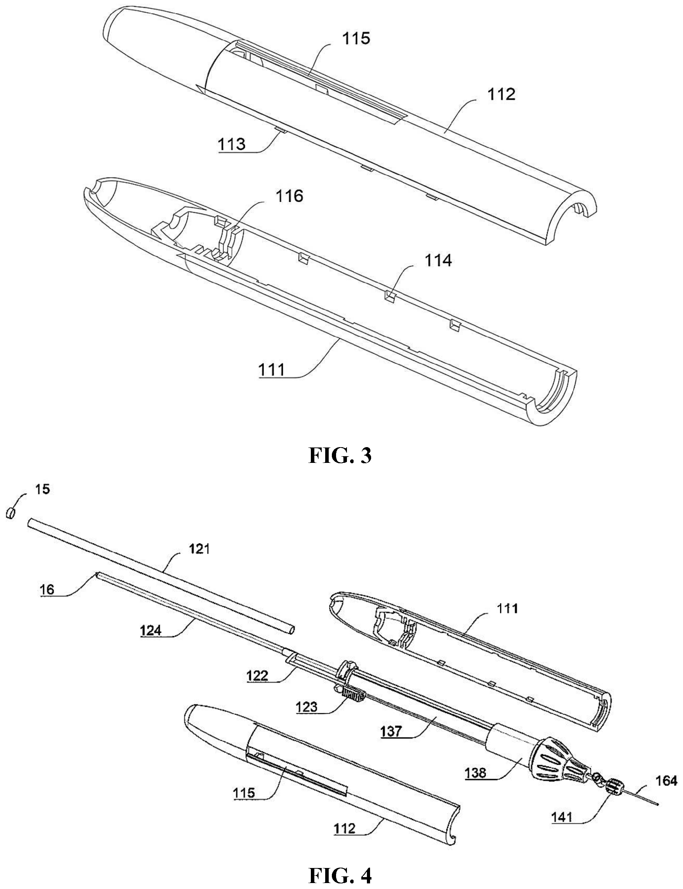

Regarding the handle housing 11, with reference to FIG. 3, the handle housing 11 is the main external part of the ligation device 1, playing a role in protecting and fixing other components and providing convenient grip for an operator. The handle housing 11 consists of an upper housing 111 and a lower housing 112. Multiple handle slots 114 are disposed at edges of two sides of the upper housing 111. Handle buckles 113 corresponding in number and position to the handle slots 114 are disposed at edges of two sides of the lower housing 112. The upper housing 111 and the lower housing 112 are detachably connected to each other via the buckles 113 and the slots 114. A strip-shaped slide rail groove 115 is formed on the surface of the lower housing 112 for guiding and limiting the pushing assembly 12. In addition, a limiting groove 116 is disposed at the front end of the handle housing 11 for fixing a fixed guide rail 136 of the first tightening assembly 13 described below.

Regarding the pushing assembly 12, with reference to FIG. 4, the pushing assembly 12 includes an outer sheath tube 121, a sliding member 122, and a pushing button 123. The outer sheath tube 121 is connected to the front end of the handle housing 11. The mesh pouch 21 is disposed at the front end of the inner cavity of the outer sheath tube 121. An inner catheter 124 is disposed inside the outer sheath tube 121. The inner catheter 124 serves as a guiding structure when the outer sheath tube 121 moves. The inner catheter 124 is fixed at the front end of the handle housing 11. The rear end of the outer sheath tube 121 is connected to the front end of the sliding member 122. The rear end of the sliding member 122 is connected to the pushing button 123. The pushing button 123 is slidable within a button slide groove of the handle housing 11. Through forward and backward movement of the pushing button 123, the sliding member 122 moves the outer sheath tube 121 forward or backward, thereby releasing or retracting the mesh pouch 21. The outer sheath tube 121 and the sliding member 122 are fixed at the front end of the handle housing 11. The fixation may be achieved by welding or other suitable methods. Optionally, the outer sheath tube 121 is rigid or flexible, pre-bent at an angle in rigid or flexible form, or controllably bendable in flexible form. The front end opening of the outer sheath tube 121 is sealed by a plug 15. Optionally, the plug 15 adopts a Transfer Interface Protector (TIP) head. In this embodiment, before operation, the front end opening of the outer sheath tube 121 is sealed by the TIP head.

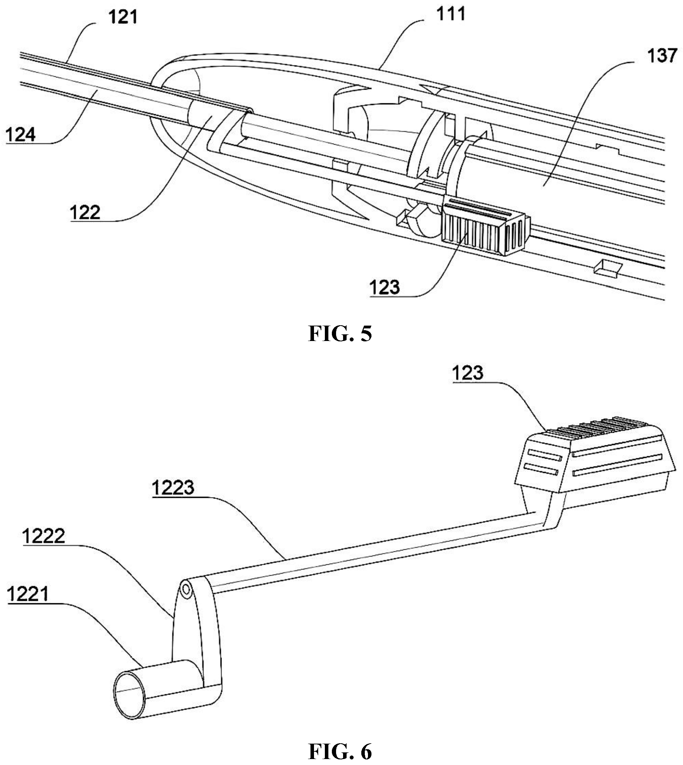

With reference to FIG. 5 and FIG. 6, the pushing button 123 and the sliding member 122 are connected through a round shaft 1223 by anchoring connection methods such as adhesive bonding or welding fixation. In this embodiment, the front end of the sliding member 122 is a connecting sleeve 1221, and the rear end of the sliding member 122 is a connecting block 1222. The connecting sleeve 1221 is connected to the rear end of the pushing tube. The connecting block 1222 protrudes from the surface of the connecting sleeve 1221 and forms a height difference. The bottom of the connecting block 1222 is connected to and integrally formed with the connecting sleeve 1221. The top of the connecting block 1222 is connected to the front end of the round shaft 1223. The pushing button 123 is connected to the rear end of the round shaft 1223.

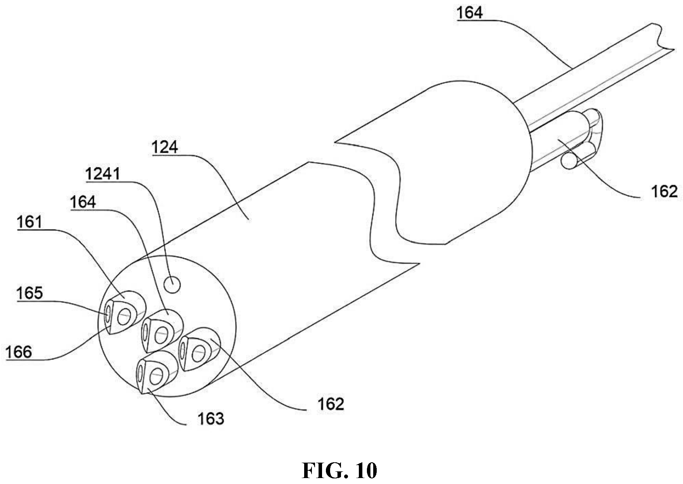

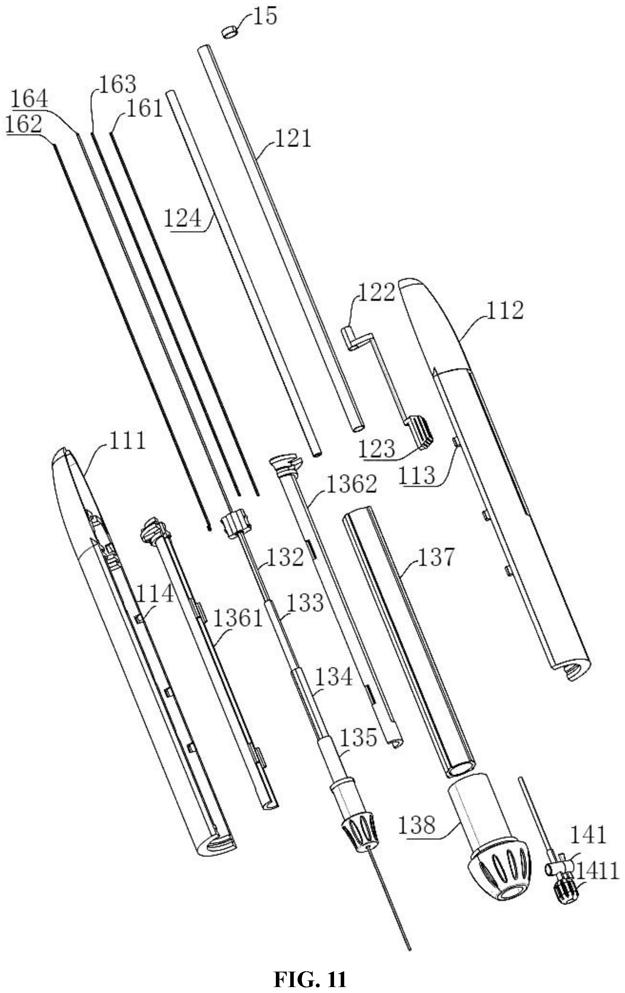

Regarding the connecting rod 16, with reference to FIG. 10 and FIG. 11, in the embodiment of this application, the two ends of the base tightening tether 223 of the base sleeve 22 and the two ends of the waist tightening tether 231 of the waist sleeve 23 are connected to the connecting rod 16. In some embodiments, the connection is achieved by anchoring methods such as knotting fixation of ropes and loops, or buckle fixation. In some embodiments, the end of the connecting rod 16 connected to the mesh sleeve structure 2 is pre-bendable to a certain angle, and the angle ranges from 0° to 90°. In this embodiment, the connecting rod 16 consists of four metal rods, including two fixed metal connecting rods 16 (that is, a first fixed rod 161 and a second fixed rod 163) and two movable metal connecting rods 16 (that is, a first movable rod 162 and a second movable rod 164). The two fixed metal connecting rods 16 are fixedly connected to the inner catheter 124 inside the outer sheath tube 121. A threading hole 165 is disposed at the front end portion of each connecting rod 16, and a cutting edge 166 is disposed at the middle position of each threading hole 165. The cutting edge 166 is formed on one-quarter of the circumference on the left and right sides of the four threading holes 165, and a thin layer of medical wax is applied to the blade edge to prevent premature cutting of the sleeve tether.

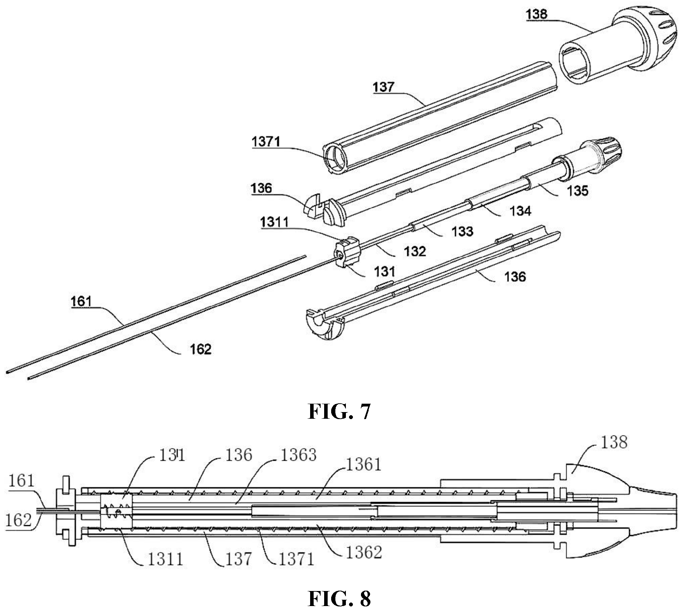

Regarding the first tightening assembly 13, with reference to FIG. 7, the first tightening assembly 13 includes a first fixed rod 161, a first movable rod 162, a guide rail slider 131, a fixed guide rail 136, a threaded guide sleeve 137, and a tail knob 138.

The two ends of the base sleeve 22 are connected to the first fixed rod 161 and the first movable rod 162, respectively. The first fixed rod 161 is fixed inside the inner catheter 124 at the front end of the handle housing 11. The first movable rod 162 is connected to the guide rail slider 131. The fixed guide rail 136 is normally fixedly mounted inside the handle housing 11. Specifically, the fixed guide rail 136 is fixedly connected to the handle housing 11. In some embodiments, the fixed guide rail 136 may be fixedly connected to the handle housing 11 through adhesive bonding, screw fixation, or buckle fixation. In the embodiment of this application, the fixed guide rail 136 and the handle housing 11 are constrained by the buckle 113. A limiting groove 116 is disposed at the front end of the handle housing 11. The limiting groove 116 constrains the four degrees of freedom (that is, up, down, front, and rear) of the fixed guide rail 136. A concave-convex structure constrains the left and right degrees of freedom to prevent rotation. External threads 1311 are disposed on the upper and lower sides of the guide rail slider 131, and protrusions are disposed on the left and right sides. The guide rail slider 131 is slidable in the grooves 1363 on two sides of the fixed guide rail 136 through the protrusions on the left and right sides. A threaded guide sleeve 137 is sleeved outside the fixed guide rail 136, and relative rotation is allowed between the fixed guide rail 136 and the threaded guide sleeve 137. With reference to FIG. 8, external threads 1311 are disposed on the upper and lower sides of the guide rail slider 131, and internal threads 1371 are disposed on the threaded guide sleeve 137. When the threaded guide sleeve 137 is rotated, the guide rail slider 131 does not rotate with the threaded guide sleeve 137. The guide rail slider 131 is axially movable only within the grooves 1363 on two sides of the fixed guide rail 136. Movement of the guide rail slider 131 drives the first movable rod 162 to move. Movement of the first movable rod 162 tightens one end of the base tightening tether 223 of the base sleeve 22 and cooperates with the other end to tighten the front end opening 24 of the mesh pouch 21. Moreover, the tail knob 138 and the threaded guide sleeve 137 are fixedly connected in a fitted manner. Optionally, the tail knob 138 and the threaded guide sleeve 137 are connected through, for example, adhesive bonding, screw fixation, or buckle fixation. In this embodiment, a concave-convex connection is adopted between the tail knob 138 and the threaded guide sleeve 137 to constrain the four degrees of freedom (that is, front, rear, up, and down).

In addition, a multi-stage telescopic rod is connected to the rear end of the guide rail slider 131. The multi-stage telescopic rod includes a first telescopic rod 132 at the foremost stage, a second telescopic rod 133 and a third telescopic rod 134 in the middle, and a fourth telescopic rod 135 at the last stage. The multi-stage telescopic rod provides auxiliary guidance and limitation for movement of the guide rail slider 131.

In some embodiments, the four rods of the multi-stage telescopic rod are connected through fits. After every two adjacent rod bodies are connected, the four degrees of freedom (that is, up, down, left, and right) are restricted, and only front-rear movement is allowed. Optionally, every two adjacent rod bodies are radially fitted through concave-convex structures at the front and rear ends to prevent relative rotation.

In some embodiments, the fixed guide rail 136 includes an upper fixed block 1361 and a lower fixed block 1362. The upper fixed block 1361 is detachably connected to the lower fixed block 1362.

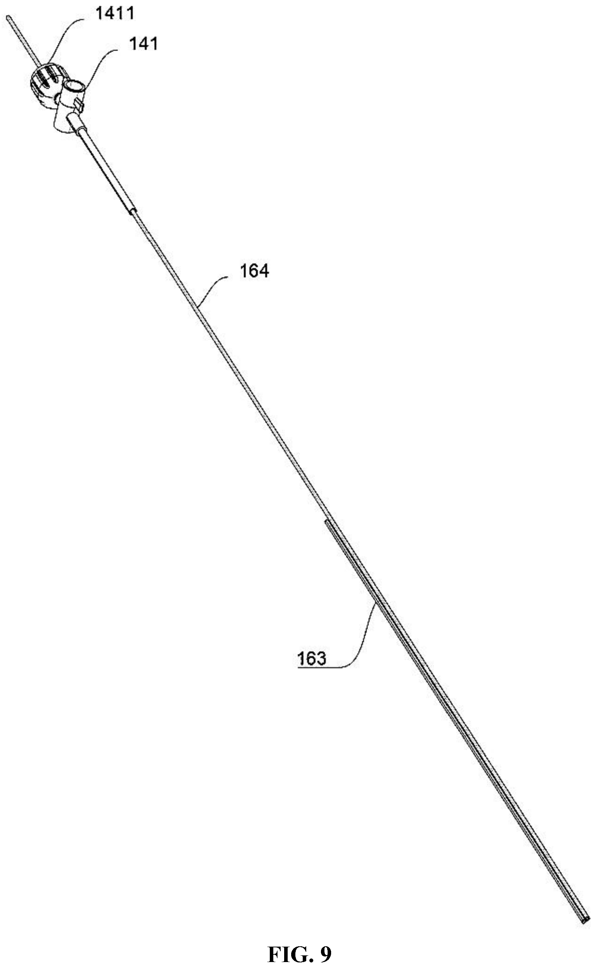

Regarding the second tightening assembly 14, with reference to FIG. 9, the second tightening assembly 14 includes a second fixed rod 163, a second movable rod 164, and a wire retraction component. The two ends of the waist tightening tether 231 of the waist sleeve 23 are connected to the second fixed rod 163 and the second movable rod 164, respectively. The second fixed rod 163 is fixed inside the inner catheter 124 at the front end of the handle housing 11. The second movable rod 164 sequentially passes through the inner catheter 124, the guide rail slider 131, and the multi-stage telescopic rod, and is connected to the wire retraction component. The wire retraction component is connected to the waist tightening tether 231 of the waist sleeve 23. In this embodiment, the second tightening assembly adopts a Luer connector 141. The Luer connector 141 is connected to the rear end of the handle housing 11. One end of the waist tightening tether 231 is connected to the Luer connector 141 through the second movable rod 164. The Luer connector 141 provides temporary fixation for the waist tightening tether 231. Moreover, a tail-end knob 1411 is disposed at the tail of the Luer connector 141. When the tail-end knob 1411 is pulled outward, the tail-end knob 1411 drives the tightening of the second movable rod 164 and the waist tightening tether 231, thereby tightening the waist of the mesh pouch 21.

Regarding the mesh sleeve structure 2, with reference to FIG. 12, the mesh sleeve structure 2 includes the mesh pouch 21 and sleeves. The sleeves include the base sleeve 22 and the waist sleeve 23. The base sleeve 22 is connected to the front end opening 24 of the mesh pouch 21 and is configured to tighten the front end opening 24 of the mesh pouch 21 for ligation of the left atrial appendage 31. The waist sleeve 23 is connected to the waist of the mesh pouch 21 and is configured to tighten the waist of the mesh pouch 21 to squeeze the waist and front portion of the left atrial appendage 31.

The mesh sleeve structure 2 will be described below.

The mesh pouch 21 is a woven polygonal structure and a three-dimensional open structure with an internal space. In some embodiments, with reference to FIG. 12 and FIG. 13, the open structure may have one open end or openings at two ends, that is, a front end opening 24 and a rear end opening 25 are both included. The front end refers to the end connected to the base sleeve 22, and the rear end refers to the end that is not connected to the base sleeve 22. The mesh sleeve structure 2 is tightened and fixed outside the left atrial appendage 31 in two tightening steps. The polygonal structure of the mesh pouch 21 can increase friction force and facilitate the covering of the left atrial appendage 31. In this embodiment, the base sleeve 22 is disposed at the front end opening 24 of the mesh pouch 21. Specifically, the base sleeve 22 is composed of a partially non-closed cavity portion 221 with certain rigidity and an elongated connecting portion 222. The partially non-closed cavity portion 221 is a C-shaped or U-shaped rigid cavity or a semi-arc cavity. The rigid cavity can ensure convenient covering of the left atrial appendage 31 by the base sleeve 22. One end of the elongated connecting portion 222 of the base sleeve 22 is connected to the middle of the cavity portion 221, and the other end is connected to a lead hole 1241 in the inner catheter 124. The elongated connecting portion 222 adopts an elongated tube. The elongated tube is configured to provide a rigid connection between the mesh sleeve structure 2 and the ligation device 1. One or multiple strands of the base tightening tether 223 extending from the front end of the mesh pouch 21 are stored inside the cavity portion 221 of the base sleeve 22. A fine slit is disposed on the side of the cavity portion 221. During the tightening process, the base tightening tether 223 disengages from the fine slit. In this embodiment, the mesh pouch 21 has a long axis of 5.5 cm and a short axis of 3 cm. The mesh pouch 21 is made of an alloy material with a memory function and certain rigidity. Optionally, for example, a nickel-titanium alloy is used.

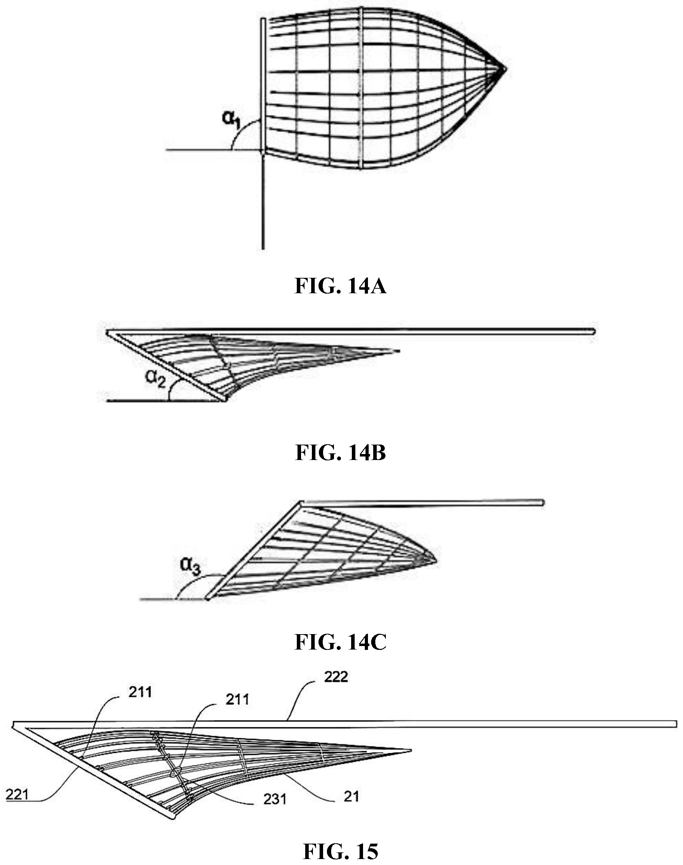

With reference to FIG. 14, in the process in which the front end opening 24 of the mesh pouch 21 is pushed to the base of the left atrial appendage 31, FIG. 14A shows the state when the mesh pouch 21 covers the base of the left atrial appendage 31, and FIG. 14B and FIG. 14C show angles of a movable range of the mesh pouch 21. Optionally, the front end opening 24 and the lower end surface of the left atrial appendage 31 may form an acute angle, a right angle, or an obtuse angle.

In this embodiment, a circle of rope loops 211 is arranged along the circumferential direction of the front end opening 24 of the mesh pouch 21. The rope loops 211 are woven integrally with the mesh pouch 21. The base tightening tether 223 sequentially pass through the rope loops 211 and are stored in the cavity portion 221.

The waist sleeve 23 differs in structure from the base sleeve 22. The waist sleeve 23 does not include a rigid cavity. The waist sleeve 23 is composed of the waist tightening tether 231 and multiple rope loops 211 woven circumferentially at the waist of the mesh pouch 21. The material of the waist tightening tether 231 of the waist sleeve 23 is consistent with the woven material of the mesh pouch 21. With reference to FIG. 15, multiple rope loops 211 are woven at both the base and the waist of the mesh pouch 21. The tightening tethers of the base sleeve 22 and the waist sleeve 23 are connected to the mesh pouch 21 by passing through the rope loops 211. Optionally, the number of waist sleeves 23 has no limit as long as conditions permit. A greater number of waist sleeves 23 produces a better tightening effect on the left atrial appendage 31 and makes expulsion of internal blood easier.

In some embodiments, the tightening tethers of the base sleeve 22 and the waist sleeve 23 adopt soft rope structures made of polymer or metal materials, optionally high-molecular-weight polyethylene, nickel-titanium, or other materials. The waist tightening tether 231 of the waist sleeve 23 restricts six degrees of freedom through torsional friction. After the waist tightening tether 231 of the waist sleeve 23 is released, freedom is regained through torsion to achieve temporary fixation. Rotation of the Luer connector 141 temporarily fixes the waist tightening tether 231.

The tissue ligation system provided by this application can not only ligate a neoplastic object or soft tissue in a body but also contract or squeeze to expel excess blood inside the neoplastic object or soft tissue, thereby preventing secondary thrombosis, accelerating atrophy and endothelialization of the neoplastic object or soft tissue, and promoting postoperative recovery.

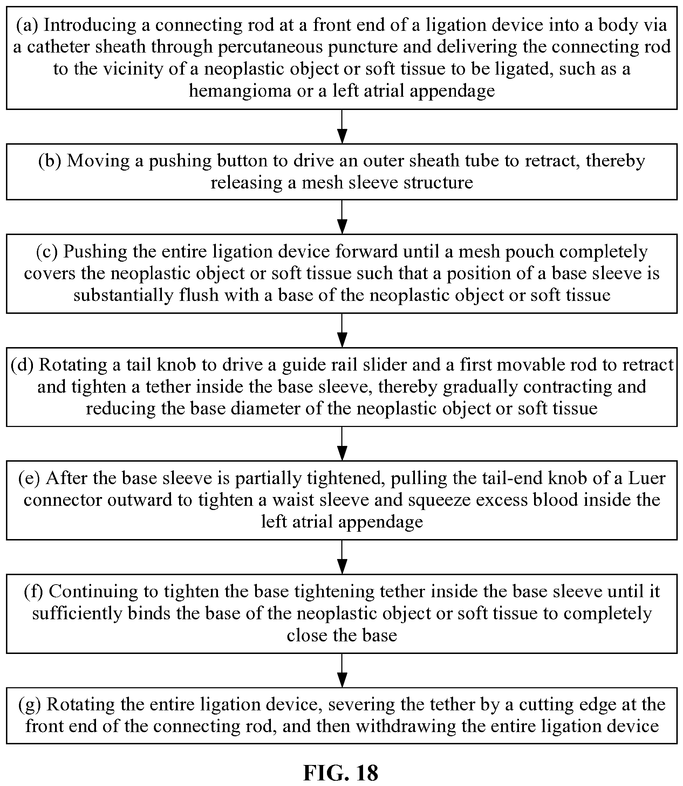

With reference to FIG. 18, this application provides a method of using the preceding tissue ligation system. The method includes steps (a) to (g).
(a) A connecting rod 16 at a front end of a ligation device 1 is introduced into a body via a catheter sheath through percutaneous puncture and is delivered to the vicinity of a neoplastic object or soft tissue to be ligated, such as a hemangioma or a left atrial appendage.
(b) A pushing button 123 is moved to drive an outer sheath tube 121 to retract, thereby releasing a mesh sleeve structure 2.
(c) The entire ligation device 1 is pushed forward until a mesh pouch 21 completely covers the neoplastic object or soft tissue such that a cavity portion 221 of a base sleeve 22 is substantially flush with a base of the neoplastic object or soft tissue.
(d) A tail knob 138 is rotated to drive a guide rail slider 131 and a first movable rod 162 to retract and tighten a base tightening tether 223 inside the cavity portion 221, thereby gradually contracting and reducing the base diameter of the neoplastic object or soft tissue.
(e) After the base tightening tether 223 of the base sleeve 22 is partially tightened, a tail-end knob 1411 of a Luer connector 141 is pulled outward to tighten the waist tightening tether 231 of the waist sleeve 23 and squeeze excess blood inside the left atrial appendage 31.
(f) The base tightening tether 223 inside the cavity portion 221 of the base sleeve 22 continues to be tightened until the base tightening tether 223 fully tightens the base of the neoplastic object or soft tissue and completely closes the base.
(g) The entire ligation device 1 is rotated. A cutting edge 166 at the front end of the connecting rod 16 severs the base tightening tether 223 and the waist tightening tether 231. The entire ligation device is then withdrawn.

During operation of the ligation system of this application, when the mesh pouch 21 ligates the left atrial appendage 31, the tether of the base sleeve 22 is first tightened. After the tether of the base sleeve 22 is tightened to 90% to 95%, the tether of the waist sleeve 23 is tightened to expel blood inside the left atrial appendage 31, thereby preventing secondary thrombosis and facilitating rapid atrophy of the left atrial appendage 31. Then, the tether of the base sleeve 22 is tightened to 100% to completely occlude the left atrial appendage 31. The tightening method of the tethers may optionally be tightening and fixation in a form of a one-way zip tie, knotting fixation, or slide buckle/fastener fixation, including but not limited to a sliding tightening and fixation method. In the embodiment of this application, the tether is woven into a one-way slidable rope knot. One end of the tether is fixed with constraint, and the other end is pulled to slide toward the distal end to achieve tightening. Optionally, the exposed external tether is covered with a reinforcement layer. Optionally, the covering method may be a sleeve, weaving, or coating, including but not limited to overmolding. Optionally, the reinforcement layer material may be a polymer material such as PTFE, ePTFE, or silicone, or a composite material such as nickel-titanium or stainless steel. Optionally, the cavity portion 221 in this application is covered with a hydrophilic coating on the inner or outer surface to facilitate the sliding of the tether.

With reference to FIG. 16 and FIG. 17, specific implementations of surgical application of the delivery system of this application are as follows: This application is suitable for advancing to the left atrial appendage 31 using minimally invasive methods, for example, through small incisions above or below the chest or through the chest, through incisions in the costal cartilage or xiphoid process, through a left subaxillary incision, through a port, or through the vascular system, or for open surgical approaches, for example, median sternotomy, mini-sternotomy, thoracotomy, or thoracoscopy. In this embodiment, a multi-lumen puncture sheath is used to puncture the epicardium. A lumen of the multi-lumen puncture sheath allows a coronary fiberoptic endoscope having an outer diameter of 2 mm to enter a pericardial layer to observe a front end of a chicken-wing shaped left atrial appendage 31. Among the tubes of the multi-lumen puncture sheath, a lumen tube may be provided for injecting a contrast agent to assist a physician in determining positions of the endoscope and the left atrial appendage 31. Subsequently, the left atrial appendage 31 ligation system of this application is advanced through a lumen of the puncture sheath via a pericardial approach. Under direct endoscopic vision, the ligation system reaches the front end of the left atrial appendage 31 to capture the left atrial appendage 31. The pushing button 123 is moved backward to retract the outer sheath tube 121 and release the mesh pouch 21 pre-loaded in the outer sheath tube 121 to the outer edge of the left atrial appendage 31. The handle is pushed forward as a whole. The mesh pouch 21 slides forward along the lower outer edge of the left atrial appendage 31. The base of the mesh pouch 21 hooks the front outer edge of the left atrial appendage 31. The rigid cavity portion 221 containing the base tightening tether 223 of the base sleeve 22 slides to the base of the left atrial appendage 31 and begins to tighten the base of the mesh pouch 21. Then, the waist tightening tether 231 of the waist sleeve 23 of the mesh pouch 21 is tightened. The system can repeatedly capture the left atrial appendage 31. If the first capture position is not ideal, the pushing button 123 is slid upward to retract the mesh sleeve structure 2. The mesh pouch 21 is released again at a suitable position. The tail knob 138 and the tail-end knob 1411 are rotated to tighten the tethers, thereby closing the left atrial appendage 31. The entire handle is slowly rotated. The cutting edges 166 on the left and right sides of the threading holes 165 of the connecting rod 16 sever one end of the tethers. The system is then withdrawn as a whole, and excess lines are cut. Final left atrial angiography is performed to verify successful ligation of the left atrial appendage 31.

The left atrial appendage 31 ligation system of this embodiment has multiple features:
One-way slidable rope knot: The tether adopts a one-way slidable rope knot that can only be tightened and cannot be loosened, which makes operation more convenient and ensures high safety.

Polygonal mesh sleeve structure 2: The mesh pouch 21 adopts a polygonal structure that increases friction force and facilitates the covering of the left atrial appendage 31 by a physician.

Tether release function: The system has a tether release function, which only requires rotation of the device, thereby avoiding multiple surgical entry points and multiple surgical instruments and reducing surgical risk.

Repeatable positioning and capture: The system can repeatedly capture the left atrial appendage 31. If the first capture position is not ideal, the physician may choose a suitable position and release the mesh sleeve again.

The left atrial appendage 31 ligation system offers multiple significant advantages in clinical application:
Single entry: No entry into the circulation is required, and management of multiple entry points is not needed, which reduces surgical complexity.

Shortest implantation time: The operation of the system is relatively simple, which makes the surgical process more efficient.

Safety: No device is left inside the left atrial appendage 31, which reduces potential complication risks.

High efficiency: The left atrial appendage 31 achieves a higher closure rate and helps prevent thrombus formation.

The beneficial effects of this application are as follows:
(1) The tissue ligation system provided by this application differs from conventional ligation devices that can only ligate tissue. Through the ligation device in this application, the mesh sleeve structure can be delivered to the left atrial appendage, and stepwise tightening is performed. The upper end and the waist of the mesh pouch are tightened separately. The mesh sleeve structure can not only surround and ligate the tissue but also squeeze and expel excess blood inside the tissue, prevent secondary thrombosis, accelerate tissue atrophy, and promote postoperative recovery.
(2) Through the tissue ligation system provided by this application, two tightenings of tissue can be achieved using only one delivery device with a single entry and exit, and occlusion can be achieved without leaving any device in the tissue, which is safe and reliable.
(3) The tissue ligation system of this application has a built-in tether release function. Only rotation of the entire handle allows the cutting edge at the front end of the rod to cut the tether, thereby achieving tether release, avoiding multiple surgical entry points and multiple surgical instruments, and reducing surgical risk.
(4) Through this application, the mesh pouch is designed into a woven polygonal structure and a three-dimensional open structure with an internal space, which can increase friction and facilitate the covering of the tissue.
(5) Through the tissue ligation system provided by this application, tissue can be repeatedly captured, and the mesh sleeve structure can be retracted by sliding the pushing button upward.

In the description of this specification, the description of reference terms "certain embodiments", "an embodiment", "some embodiments", "illustrative embodiments", "example", "specific example", or "some examples" means that specific features, structures, materials, or characteristics described in combination with the embodiment or example are included in at least one embodiment or example of this application. In the specification, the illustrative description of the preceding terms does not necessarily refer to the same embodiment or example. Moreover, the described specific features, structures, materials, or characteristics may be combined in an appropriate manner in any one or more embodiments or examples.

Moreover, terms such as "first" and "second" are used only for the purpose of description and are not to be construed as indicating or implying relative importance or implicitly indicating the number of technical features indicated. Thus, a feature defined as a "first" feature or a "second" feature may expressly or implicitly include at least one of this feature. As used herein, the term "multiple" is defined as at least two, for example, two or three, unless otherwise specified and limited.

## Claims

1. A tissue ligation system, comprising a mesh sleeve structure and a ligation device; wherein
the mesh sleeve structure comprises a mesh pouch, a base sleeve, and a waist sleeve, the base sleeve is circumferentially connected to an open end of the mesh pouch, a waist of the mesh pouch is provided with a plurality of independent small loops along one or more circumferential loops for adding one or more waist sleeves, and the waist sleeve passes through the plurality of independent small loops and is arranged along a circumferential direction of the mesh pouch;
the ligation device comprises a connecting rod and a ligation mechanism, one end of the connecting rod is connected to the ligation mechanism, and the other end of the connecting rod is connected to the mesh sleeve structure through a tether inside the connecting rod;
the ligation mechanism comprises a handle housing and a pushing assembly, a first tightening assembly, and a second tightening assembly installed on the handle housing;
the pushing assembly is connected to the mesh sleeve structure and is configured to release the mesh sleeve structure and deliver the mesh sleeve structure to a left atrial appendage;
the first tightening assembly comprises a first fixed rod, a first movable rod, and a first driving assembly, and the second tightening assembly comprises a second fixed rod, a second movable rod, and a second driving assembly;
the base sleeve comprises a base tightening tether, two ends of the base tightening tether are connected to the first fixed rod and the first movable rod, respectively, and the first driving assembly is connected to and drives the first movable rod to move, thereby driving the base tightening tether to tighten; and
the waist sleeve comprises a waist tightening tether, two ends of the waist tightening tether are connected to the second fixed rod and the second movable rod, respectively, and the second driving assembly is connected to and drives the second movable rod to move, thereby driving the waist tightening tether to tighten.

2. The tissue ligation system according to claim 1, wherein the first driving assembly comprises a guide rail slider, a fixed guide rail, a threaded guide sleeve, and a tail knob; and
the first fixed rod is fixed to a front end of the handle housing, the first movable rod is connected to the guide rail slider, the guide rail slider is slidably disposed in the fixed guide rail, the fixed guide rail is fixedly mounted inside the handle housing through a concave-convex structure, the threaded guide sleeve is sleeved outside the fixed guide rail, the guide rail slider is provided with an external thread, the threaded guide sleeve is provided with an internal thread, and the guide rail slider is driven to move axially in a groove on the fixed guide rail through rotation of the threaded guide sleeve; the tail knob is fixedly connected to the threaded guide sleeve, and rotation of the tail knob drives the threaded guide sleeve to rotate; and movement of the guide rail slider drives the first movable rod to move, thereby driving the base tightening tether to tighten a base of the mesh pouch.

3. The tissue ligation system according to claim 1, wherein the second driving assembly comprises a Luer connector and a tail-end knob, the second fixed rod is fixed to a front end of the handle housing, the second movable rod extends through the handle housing and is connected to the Luer connector, and the Luer connector is connected to the waist tightening tether; and
the tail-end knob is disposed at a tail of the Luer connector, the tail-end knob is connected to the second movable rod, and the tail-end knob is pulled to drive the second movable rod and the waist tightening tether to tighten, thereby achieving tightening of the waist of the mesh pouch.

4. The tissue ligation system according to claim 1, wherein the mesh pouch is a three-dimensional structure formed by cross-weaving a plurality of strands of rope or wire and having an internal space, one end of the mesh pouch is open and circumferentially connected to the base sleeve, the other end of the mesh pouch is closed or open, and the mesh pouch is woven from a polymer or metal material having elasticity or a shape memory property.

5. The tissue ligation system according to claim 1, wherein the base sleeve is an elastic hollow tube, and a material of the hollow tube is a polymer or metal material having elasticity or a shape memory property.

6. The tissue ligation system according to claim 1, wherein an end of the connecting rod connected to the mesh sleeve structure is pre-bendable to a certain angle, and the angle ranges from 0° to 90°.

7. The tissue ligation system according to claim 1, wherein at least one lumen is disposed inside the connecting rod.
